(19)

**Europäisches Patentamt**

**European Patent Office**

**Office européen des brevets**

(11) **EP 1 444 951 A1**

(12) **EUROPÄISCHE PATENTANMELDUNG**

(43) Veröffentlichungstag:
**11.08.2004 Patentblatt 2004/33**

(51) Int Cl.[7]: **A61B 5/12**, A61B 5/0484,
G06F 17/00

(21) Anmeldenummer: **03388010.5**

(22) Anmeldetag: **07.02.2003**

(84) Benannte Vertragsstaaten:
**AT BE BG CH CY CZ DE DK EE ES FI FR GB GR
HU IE IT LI LU MC NL PT SE SI SK TR**
Benannte Erstreckungsstaaten:
**AL LT LV MK RO**

(71) Anmelder: **Ekkehard Stürzebecher
15370 Petershagen (DE)**

(72) Erfinder: **Ekkehard Stürzebecher
15370 Petershagen (DE)**

(74) Vertreter: **Christensen, Mikael T.
C/O Oticon A/S
Strandvejen 58
2900 Hellerup (DK)**

(54) **Testgerät zum objektiven Nachweis von "Auditory Steady-State Responses" (ASSR) im Frequenzbereich**

(57) Die Erfindung betrifft ein statistisches Testverfahren zum objektiven Nachweis von Auditory Steady-State Responses (ASSR) im Frequenzbereich unter Verwendung eines g-sample uniform scores Test, wobei nur die Phasenwinkel verwendet werden. In einem Ausführungsbeispiel werden die über eine Fouriertransformation berechneten Phasenwinkel verwendet. In einem anderen Ausführungsbeispiel werden spektrale Amplituden und Phasenwinkel verwendet, die Phasenwinkel aber ungerankt, während für die spektralen Amplituden weiterhin die Ranks in den Test eingehen. In einem noch anderen Ausführungsbeispiel werden die mittels der Fouriertransformation berechneten Werte der Phasenwinkel als auch der spektralen Amplituden direkt (ungerankt) verwendet. Die Erfindung betrifft noch ein Testgerät für die Ausführung der statistischen Testverfahren.

Fig. 1

Printed by Jouve, 75001 PARIS (FR)

**Beschreibung**

Die Erfindung betrifft das Gebiet der objektiven Messung des Hörvermögens unter Nutzung von auditorisch evozierten steady-state responses (auditory steady-state responses, ASSR). Die vorgeschlagenen Verfahren zum objektiven Nachweis von ASSR im Frequenzbereich können sowohl bei einem ASSR-basierten Neugeborenen-Hörscreening als auch bei der objektiven Hörschwellenmessung auf ASSR-Basis eingesetzt werden.

Es sind zwei verschiedene Arten von ASSR bekannt:

1. click-evozierte ASSR
2. ASSR, die durch einen amplituden- oder frequenzmodulierten Dauerton evoziert werden, die auch als amplitude-modulation following response (AMFR) bezeichnet werden.

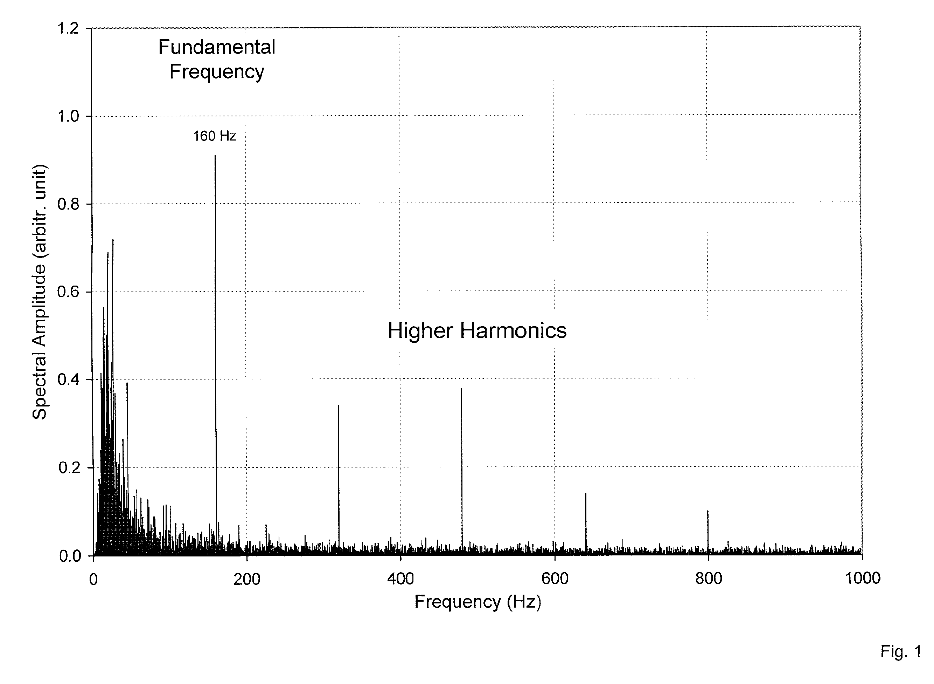

Beide Arten von ASSR werden im Frequenzbereich durch sog. Harmonische (eine Grundwelle und mehrere Oberwellen) beschrieben (Abbildung 1). Die Frequenz der Grundwelle entspricht der Click-Reizrate bzw. der Frequenz des Modulationssignals. Die Frequenzen der Oberwellen ergeben sich als Vielfache der Frequenz der Grundwelle. Das bedeutet, daß die gesamte Antwort durch wenige Spektrallinien repräsentiert wird. Der wesentliche Anteil der durch das Spontan-Elektroenzephalogramm (Spontan-EEG) bedingten Rauschleistung ist dagegen auf die zwischen den Harmonischen liegenden Spektrallinien konzentriert.

Der objektive Nachweis der bekannten ASSR erfolgt nahezu ausschließlich im Frequenzbereich. Im Frequenzbereich ist jede Spektrallinie durch eine spektrale Amplitude und einen Phasenwinkel definiert. Für den Antwort-Nachweis im Spektralbereich sind mehrere statistische Verfahren bekannt. (Stapells DR, Makeig S, Galambos R. Auditory steady-state responses: Threshold prediction using phase coherence. Electroencephalography and Clinical Neurophysiology 1987;67:260-270; Valdes JL, Perez-Abalo MC, Martin V, Savio G, Sierra C, Rodriguez E, Lins O. Comparison of statistical indicators for the automatic detection of 80 Hz auditory steady state response (AMFR). Ear and Hearing 1997;18:420-429), die als sog. One-Sample Tests nur den Phasenwinkel oder auch Phase und Amplitude einer einzelnen Spektrallinie, vorzugsweise der 1. Harmonischen (Grundwelle) auswerten. Hierzu wird das registrierte Zeitsignal epochenweise in den Frequenzbereich transformiert. Die Länge der transformierten Epochen muß so gewählt werden, daß die Epoche exakt ein ganzzahliges Vielfaches der Periodendauer der Click-Reizrate bzw. der Modulationsfrequenz ist. In dem nach der Transformation vorliegenden Frequenzspektrum wird die der Click-Reizrate bzw. der Modulationsfrequenz entsprechende Spektrallinie (Grundfrequenz) aufgesucht und getestet. Der Vorteil des Antwort-Nachweises im Frequenzbereich gegenüber dem direkten Nachweis im Zeitbereich besteht darin, daß im Spektralbereich der Anteil der Rauschleistung, der durch die zwischen den Harmonischen liegenden Spektrallinien repräsentiert wird, die Response Detection nicht stört, da diese Spektrallinien nicht in die Testung einbezogen werden. Der Nachteil der in den oben genannten Publikationen eingesetzten sogenannten "one-sample Tests" besteht in der Beschränkung der statistischen Testung auf die Grundfrequenz. ASSR werden jedoch in der Regel nicht allein durch die der Click-Folgefrequenz bzw. der Modulationsfrequenz entsprechende Grundfrequenz, sondern auch durch eine oder mehrere Oberwellen repräsentiert, auf die ein nicht zu vernachlässigender Anteil der Response-Signalleistung entfällt. Ein objektives Nachweisverfahren, das sich nur auf die Grundwelle beschränkt, ist deshalb nicht optimal.

Von Stürzebecher sowie von Stürzebecher et al. werden ebenfalls im Frequenzbereich arbeitende statistische TestVerfahren beschrieben (Stürzebecher E: "Method for hearing screening of newborn by means of steady-state response evoked with high click rate", European patent application EP 01610060.4.; Stürzebecher, E, Cebulla M, Baag M, Thie R: "Verfahren zur objektiven frequenzspezifischen Hörschwellenbestimmung mittels der Amplitude-Modulation Following Response (AMFR)", European patent application EP1099408 A2). Als statistische Tests werden sogenannte q-sample Tests eingesetzt, die für den statistischen Nachweis der ASSR sowohl die Grundwelle als auch die relevanten Oberwellen einbeziehen. Es handelt sich dabei um den aus der Literatur bekannten "q-sample uniform scores Test" (Mardia KV. Statistics of directional data. Academic Press London and New York 1972) und eine von Stürzebecher et al. vorgeschlagene Modifikation dieses Tests (Stürzebecher, E, Cebulla M, Baag M, Thie R: "Verfahren zur objektiven frequenzspezifischen Hörschwellenbestimmung mittels der Amplitude-Modulation Following Response (AMFR)", European patent application EP1099408 A2).

Die Rechenvorschrift für den von Mardia, 1972 beschriebenen q-sample uniform scores Test lautet:

Let $\{x_{ik}; 1 \leq i \leq m, 1 \leq k \leq q\}$ be a collection of random variables (phase angles $\varphi_{ik}$); q is the number of samples (spectral lines) with the sample size m (number of epochs), i.e. there are q x m = n phase angle values.

The n phase values were ranked in a single sequence. Let $r_{ik}$, i = 1,..., m, be the ranks of the phase angles in the *k*th sample.

The phase angles $\varphi_{ik}$ are then replaced by the uniform scores

$$\beta_{ik} = \frac{2 \cdot \pi \cdot r_{ik}}{n} \ .$$

The test statistics used is

$$W = \frac{2}{m} \cdot \sum_{k=1}^{q} \left( C_k^2 + S_k^2 \right)$$

with

$$C_k = \sum_{i=1}^{m} \cos \beta_{ik} ; \qquad S_k = \sum_{i=1}^{m} \sin \beta_{ik}$$

where
$r_{ik}$ are the ranks of the n phase angles (n = q x m),
q is the number of samples (number of included spectral lines)
and m is the sample size (number of epochs).
W is distributed as Chi-square with 2(q-1) degrees of freedom.

Wie aus der Rechenvorschrift erkennbar, werden nur die Phasenwinkel genutzt, die spektralen Amplituden bleiben unberücksichtigt. Zu diesem Informationsverlust kommt noch ein weiterer hinzu. Es gehen nicht die Phasenwinkel selber, sondern nur die Ranks in die Berechnung des Testwertes ein. Das hat zwar den Vorteil, daß das Verfahren verteilungsunabhängig (nonparametric) ist, die Folge der in Kauf genommenen Informationsverluste ist aber eine geringere Power des Tests. Für das Hörscreening und für die objektive Hörschwellenbestimmung sollte jedoch die Testpower so hoch wie möglich sein. Stürzebecher et al. haben deshalb eine Modifikation des Tests vorgenommen (hier als Test-Modifikation 1 bezeichnet), die neben den Phasen auch die spektralen Amplituden in Form der Ranks der Amplituden berücksichtigt:

Test-Modifikation 1

Additionally to the phase angles, the spectral amplitudes $A_{ik}$ were taken into account. Like the phase angles, the spectral amplitudes $A_{ik}$ are ranked in a single sequence: Let $a_{ik}$, i = 1,..., m be the ranks of the spectral amplitudes $A_{ik}$ in the $k$th sample. The phase angles $\varphi_{ik}$ were replaced by the uniform scores $\beta_{ik} = \frac{2 \cdot \pi \cdot r_{ik}}{n}$ .

The test statistics used for the modified q-sample uniform scores test is

$$W^{1*} = \frac{2^2}{q^2 \cdot (q+1)^2} \cdot \frac{2}{m} \cdot \sum_{k=1}^{q} \left( C_k^{*2} + S_k^{*2} \right)$$

with

$$C_k^* = \sum_{i=1}^{m} a_{ik} \cdot \cos \beta_{ik} ; \qquad S_k^* = \sum_{i=1}^{m} a_{ik} \cdot \sin \beta_{ik}$$

and

$$\beta_{ik} = \frac{2 \cdot \pi \cdot r_{ik}}{n},$$

where

$r_{ik}$ are the ranks of the n phase angles (n = q x m),

q is the number of samples

m is the sample size of the q samples and

$a_{ik}$ are the ranks of the n spectral amplitudes $A_{ik}$.

Diese bereits bekannte Testmodifikation nutzt zwar durch die Einbeziehung der spektralen Amplituden mehr Information aus als der q-sample uniform scores Test von Mardia (1972) und ist wegen der Verwendung der Ranks statt der realen Phasenund Amplitudenwerte weiterhin parameterfrei, die Arbeit mit den Ranks der Phasen und Amplituden anstelle der tatsächlichen Werte bedeutet aber weiterhin, daß die vorhandene Information nicht vollständig genutzt wird mit der Konsequenz, daß die Testpower nicht optimal ist.

Aufgabe der Erfindung ist es, verschiedene weitere Modifikationen des bekannten q-sample uniform scores Test vorzustellen, bei denen die zu Verfügung stehende Information der Phasen bzw. der Phasen und der spektralen Amplituden vollständig ausgenutzt wird.

Folgende neue Modifikationen des bekannten q-sample uniform scores Tests werden vorgeschlagen:

Test-Modifikation 2

Es werden nur die Phasenwinkeln verwendet, aber im Unterschied zum bekannten q-sample uniform scores Test von Mardia, 1972 wird nicht mit den Ranks, sondern mit den über die Fouriertransformation berechneten Phasenwinkeln gearbeitet.

Let $\{x_{ik}; 1 \le i \le m, 1 \le k \le q\}$ be a collection of random variables (phase angles $\varphi_{ik}$); q is the number of samples (spectral lines) with the sample size m (number of epochs), i.e. there are q x m = n phase angle values.

The test statistics used is

$$W^{2*} = \frac{2}{m} \cdot \sum_{k=1}^{q} \left( C_k^2 + S_k^2 \right)$$

with

$$C_k = \sum_{i=1}^{m} \cos \varphi_{ik}; \qquad S_k = \sum_{i=1}^{m} \sin \varphi_{ik}$$

where

q is the number of samples (number of included spectral lines)

and m is the sample size (number of epochs).

Test-Modifikation 3

Es werden wie bei der bekannten Modifikation spektrale Amplituden und Phasenwinkel verwendet, die Phasenwinkel aber ungerankt, während für die spektralen Amplituden weiterhin die Ranks in den Test eingehen.

The spectral amplitudes $A_{ik}$ are ranked in a single sequence:

Let $a_{ik}$, i = 1,..., m be the ranks of the spectral amplitudes $A_{ik}$ in the *k*th sample.

The test statistics used is

$$W^{3*} = \frac{2^2}{q^2 \cdot (q+1)^2} \cdot \frac{2}{m} \cdot \sum_{k=1}^{q} \left( C_k^{*2} + S_k^{*2} \right)$$

with

$$C_k^* = \sum_{i=1}^{m} a_{ik} \cdot \cos\varphi_{ik}; \quad S_k^* = \sum_{i=1}^{m} a_{ik} \cdot \sin\varphi_{ik}$$

where
q is the number of samples
m is the sample size of the q samples and
$a_{ik}$ are the ranks of the spectral amplitudes $A_{ik}$
$\varphi_{ik}$ are the phase angles.

Test-Modifikation 4

Hier werden sowohl die mittels der Fouriertransfomation berechneten Werte der Phasenwinkel als auch der spektralen Amplituden direkt (ungerankt) verwendet.
The test statistics used is

$$W^{4*} = \frac{2^2}{q^2 \cdot (q+1)^2} \cdot \frac{2}{m} \cdot \sum_{k=1}^{q} \left( C_k^{*2} + S_k^{*2} \right)$$

with

$$C_k^* = \sum_{i=1}^{m} A_{ik} \cdot \cos\varphi_{ik}; \quad S_k^* = \sum_{i=1}^{m} A_{ik} \cdot \sin\varphi_{ik}$$

where
q is the number of samples
m is the sample size of the q samples and
$A_{ik}$ are the spectral amplitudes
$\varphi_{ik}$ are the phase angles.
Ein wesentliches Problem bei der Anwendung der erfindungsgemäßen Test-Modifikationen besteht darin, daß infolge der durchgeführten Modifikationen die jeweils zugehörige Dichtefunktion der Testwerte (probability density function) für die Nullhypothese unbekannt ist. Deshalb können die für die Anwendung der Tests erforderlichen kritischen Testwerte (erforderlich für die Entscheidung: Testergebnis positiv oder negativ) nicht den in der Literatur vorliegenden Tabellen entnommen werden.
Eine bekannte Möglichkeit für die Berechnung der Dichtefunktion der Nullhypothese bietet die Monte Carlo Simulation. Hierbei wird mit einem Zufallsgenerator eine sehr große Anzahl Paare von Zufallszahlen erzeugt. Aus jedem Zahlen-Paar wird eine spektrale Amplitude und ein Phasenwinkel berechnet und darauf der statistische Test angewendet. Aus der resultierenden sehr großen Anzahl von Testwerten wird die Verteilung der Nullhypothese berechnet. Aus der Verteilung kann der gesuchte kritische Testwert abgelesen werden.
Die bei den Simulationen angenommene Normalverteilung der spektralen Amplituden und Phasen kann jedoch bei den realen spektralen Amplituden und Phasenwinkeln nicht vorausgesetzt werden.

Bestandteil der Erfindung ist deshalb das folgende Verfahren zur Berechnung der Verteilung der Nullhypothese, das die reale Verteilung der spektralen Amplituden und Phasenwinkel berücksichtigt:

Voraussetzung ist das Vorliegen einer großen Anzahl (>100) ASSR-Recordings, bei denen die Rohdaten des abgeleiteten Elektroenzephalogramms (EEG) kontinuierlich auf der Festplatte gespeichert wurden (ca. 200 Epochen, Länge einer Epoche ca. 1 Sekunde). Wie **Abbildung 1** zeigt, ist die Antwort auf wenige Spektrallinien (Grundwelle bei 160 Hz, die höheren Harmonischen bei Vielfachen von 160 Hz) begrenzt. Da bei einer Epochenlänge von etwa 1 Sekunde die spektrale Auflösung etwa 1 Hz beträgt, liegen zwischen zwei Harmonischen mehr als 150 Spektrallinien, die nur das aus dem Spontan-EEG resultierende Rauschen enthalten. Wenn man den statistischen Test auf diese Spektrallinien anwendet, so erhält man bei 100 Recording von je 200 Epochen etwa 3 000 000 Testwerte (100x200x150). Die aus diesen Testwerten berechnete Verteilung stellt eine sehr gute Schätzung der für die realen Daten zutreffenden Dichtefunktion der Nullhypothese dar, aus der der gesuchte kritische Testwert abgelesen werden kann.

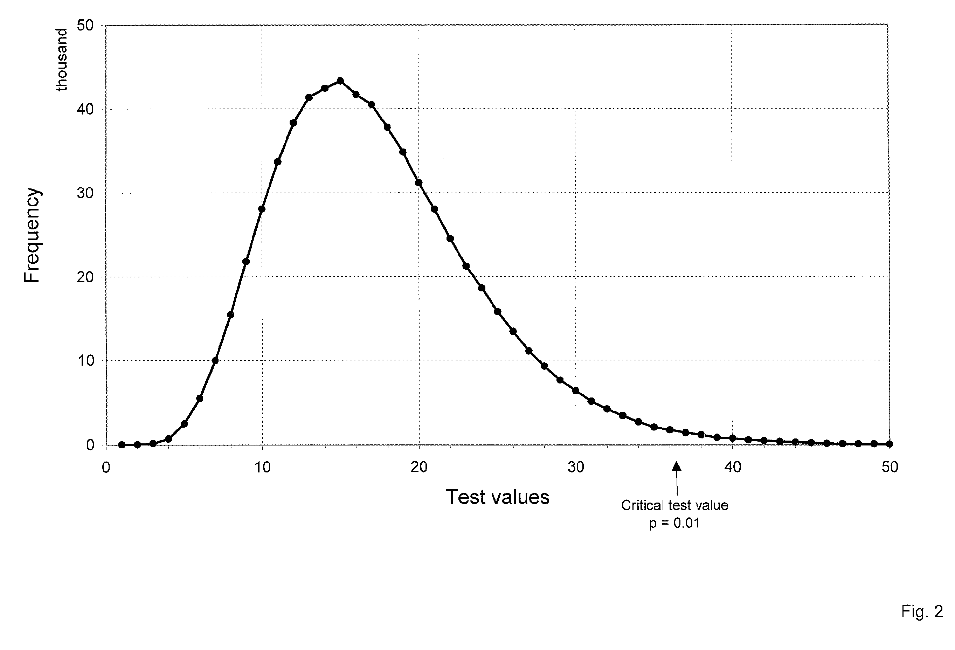

Als Beispiel ist in **Abbildung 2** die mittels der hier beschriebenen Methode berechnete Dichtefunktion der Nullhypothese für die Test-Modifikation 1 angegeben.

Die Anwendung der erfindungsgemäßen Lösung hat folgenden Vorteil:

Während die bekannten Lösungen durch Beschränkung auf die Ranks der Phasenwinkel (q-sample uniform scores Test) bzw. die Ranks der Phasenwinkel und der spektralen Amplituden (Modifikation 1) die im Spektrum enthaltene Information nur teilweise nutzen, werden durch die erfindungsgemäßen Modifikationen (Modifikation 2 - 4) mehr Information (Mod. 2 und 3) bzw. die gesamte im Spektrum enthaltene Information (Mod. 4) genutzt. Daraus resultiert eine höhere Testpower der vorgeschlagenen Modifikationen. Eine höhere Testpower führt dazu, daß bei einem Hörscreening mit vorgegebenen Reizpegel die Antwort schneller detektiert wird, dadurch ist der Zeitaufwand für das Screening geringer. Bei einer objektiven Hörschwellenbestimmung ist infolge der höheren Testpower eine genauere objektive Schwellenbestimmung möglich, da der Antwortnachweis näher an der Hörschwelle des Patienten gelingt.

Der Verzicht auf die Verteilungsunabhängigkeit bei den vorgeschlagenen Modifikationen ist kein Nachteil, da die zutreffenden Verteilungen der Nullhypothese entsprechend dem vorgeschlagenen Verfahren an Hand der Daten ermittelt werden.

**Patentansprüche**

1. Statistische Testverfahren zum objektiven Nachweis von Auditory Steady-State Responses (ASSR) im Frequenzbereich unter verwendung q-sample uniform scores Test, wobei nur die Phasenwinkeln verwendet werden.

2. Statistische testverfahren nach anspruch 1, wobei den über eine Fouriertransformation berechneten Phasenwinkeln verwendet werden.

3. Statistische Testverfahren zum objektiven Nachweis von Auditory Steady-State Responses (ASSR) im Frequenzbereich unter verwendung q-sample uniform scores Test, wobei spektrale Amplituden und Phasenwinkel verwendet, die Phasenwinkel aber ungerankt, während für die spektralen Amplituden weiterhin die Ranks in den Test eingehen.

4. Statistische Testverfahren zum objektiven Nachweis von Auditory Steady-State Responses (ASSR) im Frequenzbereich unter verwendung q-sample uniform scores Test wobei die mittels der Fouriertransfomation berechneten Werte der Phasenwinkel als auch der spektralen Amplituden direkt (ungerankt) verwendet werden.

5. Statistische Testverfahren zum objektiven Nachweis von Auditory Steady-State Responses (ASSR) im Frequenzbereich unter verwendung q-sample uniform scores Test nach einer den oben genanten ansprüche wobei den testverfahren teil eine computerprogram ist, welche auf einer lagermedium, sowie einer diskette, einer CD-ROM, einer Hard-Disk oder ähnliches gelagert ist.

6. Testgerät für die ausführung einer Statistische Testverfahren zum objektiven Nachweis von Auditory Steady-State Responses (ASSR) im Frequenzbereich unter verwendung q-sample uniform scores Test, mit einer computerprogram umfassend eine oder mehrere von den obigen genanten funktionalitäten.

Fig. 1

Fig. 2

| | **Europäisches** | **EUROPÄISCHER TEILRECHERCHENBERICHT** | Nummer der Anmeldung |
|---|---|---|---|
| | **Patentamt** | der nach Regel 45 des Europäischen Patent-übereinkommens für das weitere Verfahren als europäischer Recherchenbericht gilt | EP 03 38 8010 |

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | STÜRZEBECHER E., CEBULLA M., WERNECKE K.-D.: "Objective response detection in the frequency domain: Comparison of several q-sample tests" AUDIOLOGY & NEUROOTOLOGY, Bd. 4, Nr. 1, Januar 1999 (1999-01), Seiten 2-11, XP009013652 * das ganze Dokument * --- | 6 | A61B5/12 A61B5/0484 G06F17/00 |
| X,D | WO 02 098291 A (STUERZEBECHER EKKEHARD) 12. Dezember 2002 (2002-12-12) * Seite 5, Zeile 5 - Seite 9, Zeile 29; Ansprüche * ----- | 6 | |
| | | | **RECHERCHIERTE SACHGEBIETE (Int.Cl.7)** |
| | | | A61B G06F |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

6

Unvollständig recherchierte Patentansprüche:


Nicht recherchierte Patentansprüche:

1-5

Grund für die Beschränkung der Recherche:

Ansprüche 1-4: Artikel 52 (2)(a) EPÜ - Mathematische Methode
Anspruch 5: Artikel 52 (2)(c) EPÜ - Programm für Datenverarbeitungsanlagen

| Recherchenort | Abschlußdatum der Recherche | Prüfer |
|---|---|---|
| MÜNCHEN | 24. Juli 2003 | Ruff, C |

**EP 1 444 951 A1**

**ANHANG ZUM EUROPÄISCHEN RECHERCHENBERICHT**
**ÜBER DIE EUROPÄISCHE PATENTANMELDUNG NR.**     EP 03 38 8010

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-07-2003

| Im Recherchenbericht angeführtes Patentdokument | Datum der Veröffentlichung | Mitglied(er) der Patentfamilie | Datum der Veröffentlichung |
|---|---|---|---|
| WO 02098291 A | 12-12-2002 | WO 02098291 A2 | 12-12-2002 |

Für nähere Einzelheiten zu diesem Anhang : siehe Amtsblatt des Europäischen Patentamts, Nr.12/82

EPO FORM P0461

10